Europäisches Patentamt

European Patent Office

Office européen des brevets

(19)

(11) Numéro de publication: **0 145 690**
**A1**

## (12) DEMANDE DE BREVET EUROPEEN

(21) Numéro de dépôt: **84870110.8**

(22) Date de dépôt: **03.08.84**

(51) Int. Cl.⁴: **G 01 N 33/22**

(30) Priorité: **11.08.83 LU 84958**

(43) Date de publication de la demande: **19.06.85**
**Bulletin 85/25**

(84) Etats contractants désignés: **AT DE FR GB IT**

(71) Demandeur: **CENTRE DE RECHERCHES METALLURGIQUES CENTRUM VOOR RESEARCH IN DE METALLURGIE Association sans but lucratif, Vereniging zonder winstoogmerk Rue Montoyer, 47, B-1040 Bruxelles (BE)**

(72) Inventeur: **Lückers, John, 7, rue de Beauregard, B-4051 Plainevaux (BE)**

(74) Mandataire: **Lacasse, Lucien Emile et al, CENTRE DE RECHERCHES METALLURGIQUES Abbaye du Val-Benoît 11, rue Ernest Solvay, B-4000 Liège (BE)**

(54) **Procédé de mesure de la teneur en eau des matières chargées dans un four à cuve.**

(57) On introduit dans le four à cuve des quantités connues de matière, notamment du minerai, du coke et du vent. On détermine la composition ($H_2$, $H_2O$, CO, $CO_2$, $N_2$) et le débit des gaz issus du gueulard du four à cuve. A partir de ces valeurs, on détermine la quantité provenant de la matière enfournée et, en rapportant cette quantité d'eau aux caractéristiques de la matière enfournée, notamment à sa teneur en hydrogène, on détermine la teneur en eau de cette matière enfournée.

EP 0 145 690 A1

ACTORUM AG

0145690

- 1 -

<u>Procédé de mesure de la teneur en eau des matières chargées dans un four à cuve</u>.

La présente invention a trait à un procédé de mesure de la teneur en eau des matières chargées dans un four à cuve, comme par exemple un haut fourneau.

L'invention est applicable aux fours à cuve en général, la description ci-après étant axée sur le haut fourneau dans un souci de clarté et non dans un but restrictif.

Le fonctionnement d'un four à cuve tel qu'un haut fourneau peut être grossièrement schématisé en considérant que d'une part on introduit par le haut une charge composée de minerais et de coke,-ceux-ci sont déversés de manière à constituer des couches superposées, - et d'autre part que l'on injecte par le bas des comburants et des combustibles aptes à favoriser la réduction du minerai; au fur et à mesure que cette réaction se déroule, on recharge selon les besoins.

On comprend aisément que la conduite du haut fourneau exige que l'on connaisse aussi bien la nature chimique que l'état physique (teneur en eau, granulométrie,...) des divers éléments qui y sont introduits.

En outre, il va de soi que la régulation des opérations du haut fourneau est d'autant plus fiable que la précision des données que l'on possède sur les caractéristiques des produits chargés est meilleure.

A l'heure actuelle, un des problèmes qui se pose est de déterminer avec la plus grande exactitude possible la teneur en eau des matières enfournées et en particulier celle du coke.

Parmi les méthodes de mesure existantes, les plus courantes peuvent être classées en deux grandes catégories : d'une part celles faisant appel aux sondes à rétrodiffusion de neutrons et d'autre part celles basées sur les infrarouges.

Les appareils de la première catégorie, c'est-à-dire à sonde à rétrodiffusion de neutrons, sont généralement placés au niveau des trémies peseuses par lesquelles la matière passe avant d'être prise en charge par un dispositif qui l'élève jusqu'au sommet du haut fourneau.

Dans le cas particulier du coke, l'imprécision atteint plus ou moins 1 % sinon plus, ce qui est grand si l'on sait que l'humidité du coke ne varie souvent que de 2 à 8 %. Les mesures sont faussées d'une part par le fait que ces appareils réagissent par mesure de tout l'hydrogène contenu dans la matière et sont donc influencés non seulement par l'eau, mais aussi par les

3.-

carbonates, les hydrocarbures, les matières volatiles... présents dans la charge examinée, et d'autre part du fait que la mesure est opérée dans la trémie peseuse, donc doit être faite très rapidement, car la charge de coke n'y reste pas longtemps; d'où il en résulte un manque de temps de comptage c'est-à-dire une erreur due à la nature statistique de la mesure.

La seconde catégorie comprend les appareils qui mettent en oeuvre le principe des capteurs à infrarouges. Ces capteurs sont disposés de manière à examiner la matière, par exemple quand celle-ci est transportée sur des courroies, et procèdent à la mesure en captant les rayons réfléchis par celle-ci. L'imprécision atteint 1,5 % sinon plus. La mesure est fort influencée, et donc dépendante, de la granulométrie, la couleur, la surface apparente,... et ne mesure en fait que l'humidité de surface.

On sait qu'à l'heure actuelle, les procédés de travail au haut fourneau et aussi au four à cuve en général mettent en oeuvre divers dispositifs qui analysent non seulement les produits de la charge (coke, minerais,...) et les éléments injectés (vent, hydrocarbures, charbon,...), mais aussi les produits sortant du haut fourneau comme les gaz captés au gueulard.

Le demandeur a constaté un effet inattendu, à savoir qu'en combinant judicieusement diverses opérations de mesure des caractéristiques des produits entrant et sortant du haut fourneau, il était possible d'en déduire la teneur en eau des matières chargées par exemple du coke, sans que cette dernière ne fasse l'objet d'une mesure directe.

Cette observation est à l'origine du procédé de la présente invention; ce procédé ne présentant pas les inconvénients signalés plus haut et donnant une mesure de précision supérieure à celle des deux méthodes précitées.

Le procédé de mesure de la teneur en eau des matières enfournées dans un four à cuve tel que par exemple un haut fourneau, objet de la présente invention, est essentiellement caractérisé en ce qu'on introduit une quantité connue de matière dans le four à cuve, en ce qu'on analyse les gaz issus du gueulard du four à cuve précité et en ce qu'on utilise les résultats de cette analyse pour calculer la valeur de la teneur en eau de la matière enfournée.

Il va de soi que l'on se place dans les conditions de travail normales d'un four à cuve, par exemple un haut fourneau, c'est-à-dire équipé des dispositifs habituels destinés à fournir des renseignements sur le minerai, le coke, le vent, les combustibles, l'analyse de gaz captés au gueulard, etc...,mais dépourvu éventuellement d'une mesure directe de la teneur en eau des matières enfournées, par exemple du coke, mesure qui serait moins précise que la valeur donnée par le procédé ci-dessus.

Suivant une première modalité de réalisation du procédé de la présente invention, la mesure de la teneur en eau de la matière enfournée est déduite de la variation d'une grandeur proportionnelle à la teneur en eau des gaz captés au gueulard du four à cuve, éventuellement la mesure de celle-ci.

Suivant une autre modalité de réalisation, on suit l'évolution de la teneur en eau des gaz captés au gueulard du four à cuve avant, pendant et après l'introduction de la charge de matière.

Suivant une mise en oeuvre préférentielle de la modalité précédente dans laquelle on enregistre, en continu ou non, une grandeur proportionnelle à la teneur en eau des gaz captés au gueulard du four à cuve ou la mesure de celle-ci, on estime la variation de la grandeur enregistrée par surfaçage graphique, analyse numérique ou toute autre méthode; on déduit la teneur en eau des gaz sortant du gueulard, par calcul ou comparaison par **exemple** avec des tables de données ou par toute autre méthode; on détermine le débit des gaz précités; on combine les valeurs obtenues ci-avant (teneur en eau, débit et analyse du gaz de gueulard), de manière à définir la quantité d'eau correspondant à la matière enfournée et, en rapportant cette quantité d'eau aux caractéristiques de la matière enfournée, notamment à sa teneur en hydrogène, on déduit la teneur en eau de cette matière. En particulier, le débit des gaz sortant du gueulard peut être déterminé soit par une mesure directe, soit en établissant un bilan d'azote à partir de l'analyse de ces gaz et du débit de vent introduit dans le four à cuve.

Suivant une quatrième modalité de réalisation, avant de procéder à l'analyse d'un échantillon du gaz capté au gueulard du four à cuve en vue de déterminer sa teneur en eau, on dilue cet échantillon par mélange à un gaz inerte ou non, par exemple de l'azote.

0145690

Revendications.

1. Procédé de mesure de la teneur en eau des matières enfournées dans un four à cuve tel que par exemple un haut fourneau, caractérisé en ce qu'on introduit une quantité connue de matière dans le four à cuve, en ce qu'on analyse les gaz issus du gueulard du four à cuve précité et en ce qu'on utilise les résultats de cette analyse pour calculer la valeur de la teneur en eau de la matière enfournée.

2. Procédé de mesure suivant la revendication 1, caractérisé en ce que la mesure de la teneur en eau de la matière enfournée est déduite de la variation d'une grandeur proportionnelle à la teneur en eau des gaz captés au gueulard du four à cuve, éventuellement la mesure de celle-ci.

3. Procédé de mesure suivant la revendication 2, caractérisé en ce qu'on suit l'évolution de la teneur en eau des gaz captés au gueulard du four à cuve avant, pendant et après l'introduction de la charge de matière.

4. Procédé de mesure suivant la revendication 3, dans laquelle on enregistre, en continu ou non, une grandeur proportionnelle à la teneur en eau des gaz captés au gueulard du four à cuve ou la mesure de celle-ci, caractérisé en ce qu'on estime la variation de la grandeur enregistrée par surfaçage graphique, analyse numérique ou toute autre méthode; en ce qu'on déduit la teneur en eau des gaz sortant du gueulard, par calcul ou comparaison par exemple avec des tables de données ou par toute autre méthode; en ce qu'on détermine le débit des gaz précités; en ce qu'on combine les valeurs obtenues ci-avant (teneur en eau, débit et analyse du gaz de gueulard), de manière à définir la quantité d'eau correspondant à la matière enfournée et en ce qu'en rapportant cette quantité d'eau aux caractéristiques de la matière enfournée, notamment à sa teneur en hydrogène, on déduit la teneur en eau de cette matière.

5. Procédé de mesure suivant l'une ou l'autre des revendications 1 à 4, caractérisé en ce qu'avant de procéder à l'analyse d'un échantillon du gaz capté au gueulard du four à cuve en vue de déterminer sa teneur en eau, on dilue cet échantillon par mélange à un gaz inerte ou non, par exemple de l'azote.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | GB-A-1 246 805  (COAL INDUSTRY) | | G 01 N  33/22 |
| A | GB-A- 867 811  (STOCKHAUSEN) | | |
| A | US-A-3 618 368  (LESEMANN) | | |
| A | US-A-4 002 421  (J.R. SUMMER) | | |

---

**DOMAINES TECHNIQUES RECHERCHES (Int. Cl.4)**

G 01 N
F 27 B

Le présent rapport de recherche a été établi pour toutes les revendications

| Lieu de la recherche | Date d'achèvement de la recherche | Examinateur |
|---|---|---|
| LA HAYE | 29-11-1984 | BINDON C.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82